(19) Europäisches Patentamt
European Patent Office
Office européen des brevets

(11) Veröffentlichungsnummer: **0 427 957 B1**

# (12) EUROPÄISCHE PATENTSCHRIFT

(45) Veröffentlichungstag der Patentschrift: **11.01.95**

(21) Anmeldenummer: **90119363.1**

(22) Anmeldetag: **09.10.90**

(51) Int. Cl.$^6$: **C07D 319/06**, C09K 19/12, C09K 19/34, C09K 19/30, C07C 15/14, C07C 25/24, C07C 13/19

## (54) Alkinyl-substituierte Flüssigkristallkomponenten.

(30) Priorität: **18.10.89 CH 3781/89**
**26.06.90 CH 2146/90**

(43) Veröffentlichungstag der Anmeldung:
**22.05.91 Patentblatt 91/21**

(45) Bekanntmachung des Hinweises auf die Patenterteilung:
**11.01.95 Patentblatt 95/02**

(84) Benannte Vertragsstaaten:
**CH DE FR GB IT LI NL**

(56) Entgegenhaltungen:
**EP-A- 0 122 389**
**GB-A- 2 111 992**

(73) Patentinhaber: **F. HOFFMANN-LA ROCHE AG**
**Postfach 3255**
**CH-4002 Basel (CH)**

(72) Erfinder: **Buchecker, Richard, Dr.**
**Felsenstrasse 10**
**CH-8008 Zürich (CH)**
Erfinder: **Schadt, Martin, Dr.**
**Liestalerstrasse 77**
**CH-4411 Seltisberg (CH)**
Erfinder: **Seils, Frank, Dr.**
**Bündenfeldstrasse 10**
**W-7861 Wieslet (DE)**

(74) Vertreter: **Cottong, Norbert A. et al**
**Grenzacherstrasse 124**
**Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Verbindungen mit endständigem 1-Alkinylrest, deren Herstellung, flüssigkristalline Gemische, die solche Verbindungen enthalten sowie die Verwendung für elektrooptische Zwecke.

Flüssige Kristalle werdem vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten sie bei den üblichen Betriebstemperaturen in einem möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine positive dielektrische Anisotropie und eine möglichst geringe elektrische Leitfähigkeit aufweisen.

Um diese zum Teil widersprüchlichen Anforderungen zu erfüllen, müssen in der Regel Mischungen mit bis zu etwa 15 Komponenten hergestellt werden. Es ist daher wichtig, dass die Komponenten untereinander gut mischbar sind und eine ausreichende Löslichkeit aufweisen.

Um ausreichend breite Mesophasenbereiche zu erzielen, müssen den Mischungen meist klärpunktserhöhende Komponenten zugesetzt werden, welche jedoch die Viskosität und die elektro-optischen Eigenschaften nachteilig beeinflussen können. Ferner ergeben Materialien mit niedriger optischer Anisotropie, welche z.B. für aktiv adressierte Flüssigkristallanzeigen von Interesse sind, häufig smektische Tendenzen und führen meist auch zu einer Erhöhung der Schwellenspannung, der Viskosität und/oder der Ansprechzeiten. Weiterhin besitzen unpolare Materialien mit hoher optischer Anisotropie oft nur smektische Mesophasen oder gar keine flüssigkristallinen Eigenschaften. Solche unpolaren Flüssigkristallkomponenten sind beispielsweise die in GB-A-2 111 992 beschriebenen Verbindungen, welche einen endständigen 1-Alkinylrest aufweisen.

Die Erfindung betrifft Verbindungen der allgemeinen Formel

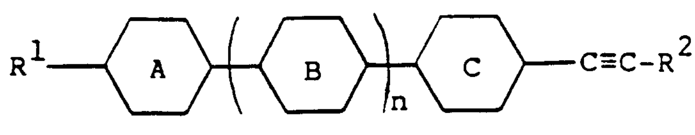

I

worin n für die Zahl O oder 1 steht; die Ringe A, B und C unabhängig voneinander trans-1,4-Cyclohexylen, trans-1,3-Dioxan-2,5-diyl oder unsubstituiertes oder fluor-substituiertes 1,4-Phenylen darstellen; $R^1$ 3E-Alkenyl mit 4 bis 12 Kohlenstoffatomen, 4-Alkenyl mit 5 bis 12 Kohlenstoffatomen oder, wenn Ring A trans-1,4-Cyclohexylen oder trans-1,3-Dioxan-2,5-diyl darstellt, auch 1E-Alkenyl mit 2 bis 12 Kohlenstoffatomen bezeichnet; und $R^2$ Wasserstoff oder $C_1$-$C_{10}$-Alkyl bedeutet.

Die erfindungsgemässen Verbindungen sind unpolare Verbindungen mit vergleichsweise sehr breiten Mesophasebereichen, hohen Klärpunkten und ausgeprägter nematischer Tendenz. Trotz der hohen Klärpunkte besitzen sie erstaunlich niedere Viskosität und günstige elektro-optische Eigenschaften, insbesondere ermöglichen sie kurze Schaltzeiten, niedrige Schwellenspannungen und je nach Wahl von $R^1$ eine Modifizierung der elastischen Eigenschaften. Ferner kann die optische Anisotropie in einem breiten Bereich variiert werden; beispielsweise besitzen die Verbindungen der Formel I, worin die Ringe A, B und C trans-1,4-Cyclohexylen und/oder trans-1,3-Dioxan-2,5-diyl bedeuten, niedrige optische Anisotropien und die Verbindungen der Formel I, worin die Ringe A, B und C unsubstituiertes oder fluorsubstituiertes 1,4-Phenylen bedeuten, besonders hohe optische Anisotropien.

Aufgrund der ausgezeichneten Eigenschaften bieten die erfindungsgemässen Verbindungen ferner die Möglichkeit, die Zahl der Komponenten im Gemisch zu verringern und damit die Gemische wesentlich zu vereinfachen. Hierbei ist von Vorteil, dass die erfindungsgemässen Verbindungen mit bekannten Materialien gut mischbar und aufgrund der niedrigen Schmelzenthalpien auch in hohen Konzentrationen gut löslich sind.

Der obige Ausdruck "fluor-substituiertes 1,4-Phenylen" umfasst im Rahmen der vorliegenden Erfindung insbesondere 2-Fluor-1,4-phenylen und 2,3-Difluor-1,4-phenylen.

Der Ausdruck "1E-Alkenyl mit 2 bis 12 Kohlenstoffatomen" umfasst geradkettige oder verzweigte Reste, insbesondere die geradkettigen Reste Vinyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undecenyl und 1E-Dodecenyl.

Der Ausdruck "3E-Alkenyl mit 4 bis 12 Kohlenstoffatomen" umfasst geradkettige oder verzweigte Reste, insbesondere die geradkettigen Reste 3-Butenyl, 3E-Pentenyl, 3E-Hexenyl, 3E-Heptenyl, 3E-Octenyl, 3E-Nonenyl, 3E-Decenyl, 3E-Undecenyl und 3E-Dodecenyl.

Der Ausdruck "4-Alkenyl mit 5 bis 12 Kohlenstoffatomen" umfasst geradkettige oder verzweigte Reste, insbesondere die geradkettigen Reste 4-Pentenyl, 4-Hexenyl, 4-Heptenyl, 4-Octenyl, 4-Nonenyl, 4-Decenyl, 4-Undecenyl und 4-Dodecenyl. Bevorzugt sind im allgemeinen 4-Pentenyl und die Z-Isomeren der weiteren, genannten Reste.

Der Ausdruck "$C_1$-$C_{10}$-Alkyl" umfasst geradkettige und verzweigte Reste, insbesondere die geradkettigen Reste Methyl, Aethyl, Propyl, Butyl, Pentyl, Hexyl, Heptyl, Octyl, Nonyl und Decyl.

Der Ausdruck "gesättigter Ring " bezeichnet im Rahmen der vorliegenden Erfindung trans-1,4-Cyclohexylen und trans-1,3-Dioxan-2,5-diyl. Der Ausdruck "aromatischer Ring" bezeichnet unsubstituiertes oder fluor-substituiertes 1,4-Phenylen, insbesondere 1,4-Phenylen, 2-Fluor-1,4-phenylen und 2,3-Difluor-1,4-phenylen.

Verbindungen der Formel I, worin $R^1$ einen geradkettigen Rest und $R^2$ Wasserstoff oder einen geradkettigen Rest bedeuten, sind bevorzugt. Gewünschtenfalls kann jedoch $R^1$ und/oder $R^2$ eine Kettenverzweigung aufweisen. Bespielsweise eignen sich Verbindungen der Formel I mit chiralem Rest $R^1$ und/oder $R^2$ als optisch aktive Dotierstoffe zur Erzielung cholesterischer Phasen.

In Formel I steht vorzugsweise höchstens einer der Ringe A, B und C (bevorzugt Ring A oder B) für trans-1,3-Dioxan-2,5-diyl.

Vorzugsweise besitzen die Verbindungen der Formel I höchstens 1 oder 2 laterale Fluorsubstituenten. Allenfalls vorhandene Fluorsubstituenten stehen vorzugsweise an Ring B oder insbesondere an Ring C.

Ein bevorzugter Aspekt betrifft diejenigen Verbindungen der Formel I, worin Ring A ein gesättigter Ring und Ring C ein aromatischer Ring ist. Im Falle von $n = 1$ kann Ring B gesättigt oder aromatisch sein.

Ein weiterer bevorzugter Aspekt betrifft diejenigen Verbindungen der Formel I, worin die Ringe A und C gesättigte Ringe darstellen. Im Falle von $n = 1$ ist vorzugsweise auch Ring B ein gesättigter Ring. Besonders bevorzugt sind diejenigen Verbindungen, worin einer der Ringe (insbesondere Ring A oder B) für trans-1,4-Cyclohexylen oder trans-1,3-Dioxan-2,5-diyl steht und die andern Ringe für trans-1,4-Cyclohexylen stehen. Diese Verbindungen besitzen sehr niedrige optische Anisotropien.

Weiterhin sind diejenigen Verbindungen der Formel I bevorzugt, worin die Ringe A und C aromatische Ringe darstellen. Im Falle $n = 1$ ist vorzugsweise auch Ring B ein aromatischer Ring. Diese Verbindungen besitzen besonders hohe optische Anisotropien.

Beispiele besonders bevorzugter Untergruppen von Verbindungen der Formel I sind die Verbindungen der allgemeinen Formeln

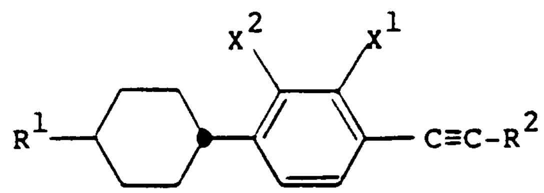

I - 1

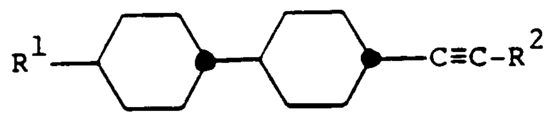

I - 2

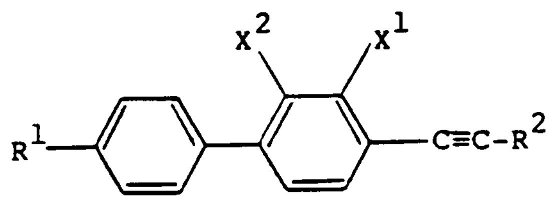

I - 3

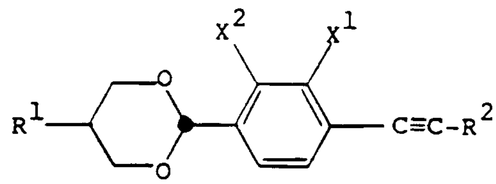

I-4

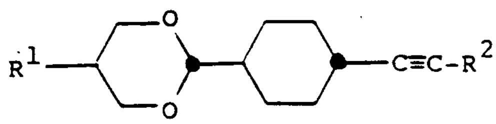

I-5

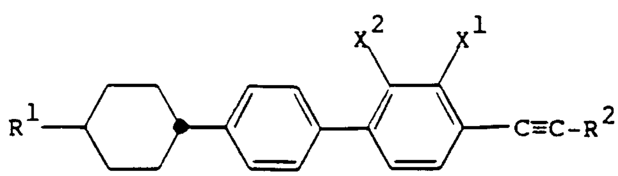

I-6

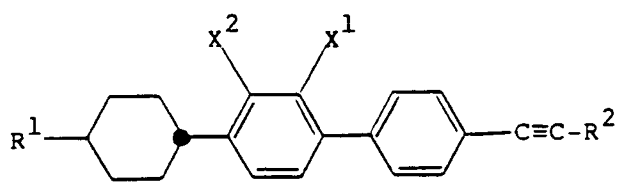

I-7

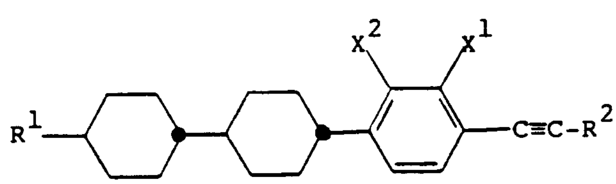

I-8

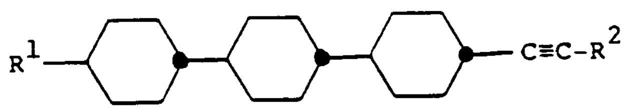

I-9

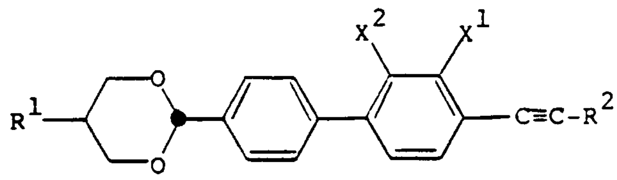

I-10

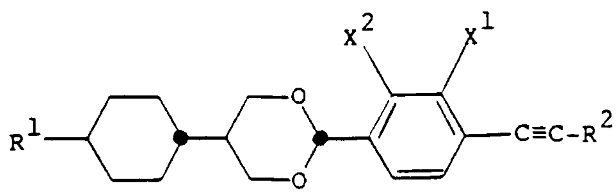

I-11

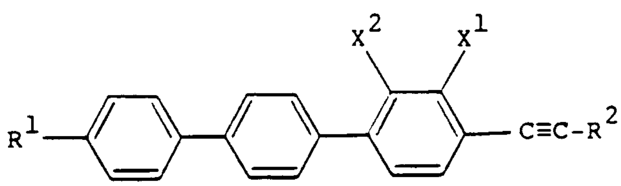

I-12

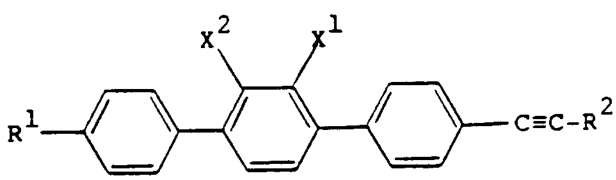

I-13

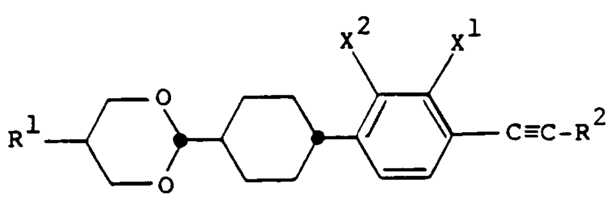

I-14

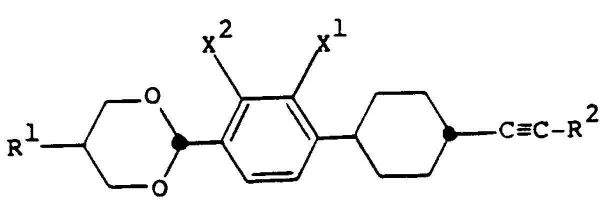

I-15

worin $X^1$ und $X^2$ unabhängig voneinander Wasserstoff oder Fluor darstellen; $R^2$ Wasserstoff oder $C_1$-$C_{10}$-Alkyl bedeutet; und $R^1$ 3E-Alkenyl mit 4 bis 12 Kohlenstoffatomen, 4-Alkenyl mit 5 bis 12 Kohlenstoffatomen oder in den Formeln I-1, I-2, I-4, I-5, I-6, I-7, I-8, I-9, I-10, I-11, I-14 und I-15 auch 1E-Alkenyl mit 2 bis 12 Kohlenstoffatomen bezeichnet.

Vorzugsweise besitzt $R^1$ in den obigen Formeln höchstens 7 Kohlenstoffatome. Besonders bevorzugt sind die Reste $R^1$ mit bis zu 5 Kohlenstoffatomen.

$R^2$ bedeutet in den obigen Formeln vorzugsweise Wasserstoff oder $C_1$-$C_5$-Alkyl, insbesondere Wasserstoff oder $C_1$-$C_3$-Alkyl.

Die Verbindungen der Formel I können erfindungsgemäss dadurch hergestellt werden, dass man

a) zur Herstellung der Verbindungen der Formel I, worin $R^2$ Wasserstoff bedeutet, eine Verbindung der allgemeinen Formel

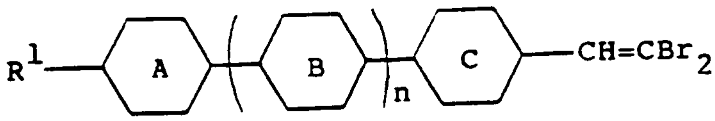

II

worin n, $R^1$ und die Ringe A, B und C die in Formel I gegebenen Bedeutungen haben, mit Alkyllithium umsetzt, oder

b) zur Herstellung der Verbindungen der Formel I, worin $R^2$ $C_1$-$C_{12}$-Alkyl bedeutet, eine Verbindung der Formel I, worin $R^2$ Wasserstoff bedeutet, alkyliert.

Die Umsetzung einer Verbindung der Formel II mit Alkyllithium kann in an sich bekannter Weise erfolgen. Bevorzugte Lithiumverbindungen sind $C_1$-$C_4$-Alkyllithium, wie Methyllithium, Butyllithium und dergleichen. Die Umsetzung erfolgt vorzugsweise in einem interten organischen Lösungsmittel, wie Hexan, Tetrahydrofuran und dergleichen, bei Temperaturen von höchstens etwa -70 °C.

Die Alkylierung der Verbindungen der Formel I, worin $R^2$ Wasserstoff bedeutet, kann nach üblichen Alkylierungsmethoden erfolgen. Nach einer bevorzugten Methode wird die Acetylenverbindung mit Base deprotoniert und dann mit $C_1$-$C_{10}$-Alkylhalogenid alkyliert. Die Deprotonierung kann beispielsweise mit Butyllithium, Methyllithium, Natriumhydrid und dergleichen in einem inerten organischen Lösungsmittel (z.B. Hexan, Tetrahydrofuran und dergleichen) bei Temperaturen unterhalb etwa -30 °C erfolgen. Der anschliessende Alkylierungsschritt kann beispielsweise mit Alkylchlorid, -bromid oder -jodid in Gegenwart von Hexamethylphosphorsäuretriamid durchgeführt werden.

Die Verbindungen der Formel II sind neu. Sie können in an sich bekannter Weise aus den Cyanoverbindungen der allgemeinen Formel

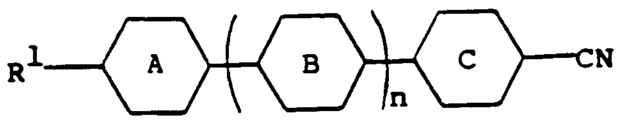

III

worin n, $R^1$ und die Ringe A, B und C die in Formel I gegebenen Bedeutungen haben, hergestellt werden. Beispielsweise kann eine Verbindung der Formel III mit Diisobutylaluminiumhydrid reduziert und der erhaltene Aldehyd mit Tetrabrommethan und Triphenylphosphin zur Verbindung der Formel II umgesetzt werden.

Die Verbindungen der Formel III sind bekannte oder Analoge bekannter Verbindungen und können nach den in EP-A-0122389, EP-A-0167912 und EP-A-0315050 beschriebenen Methoden hergestellt werden.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen und OMI-Zellen. Bevorzugte

Gemische sind daher diejenigen, welche eine oder mehrere Verbindungen der Formel I und eine oder mehrere Verbindungen mit positiver dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% oder mehr betragen. Im allgemeinen ist ein Anteil von etwa 3-50 Gew.-% insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln

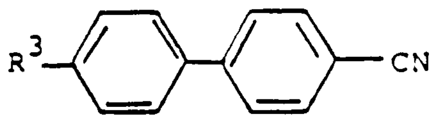

V

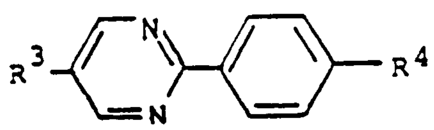

VI

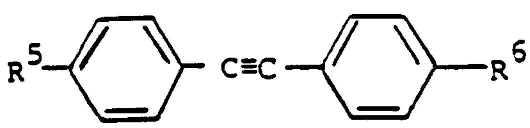

VII

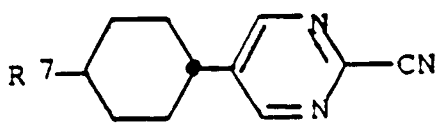

VIII

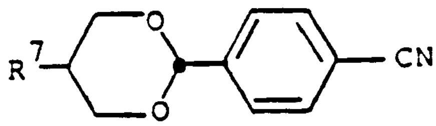

IX

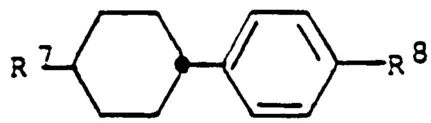

X

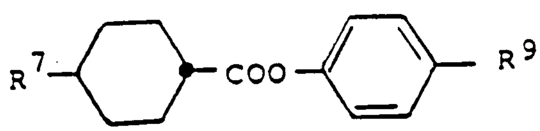

XI

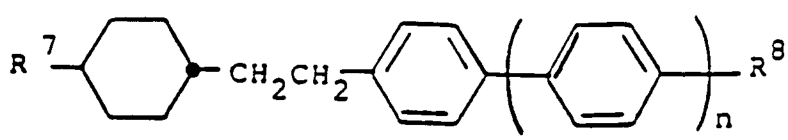

XII

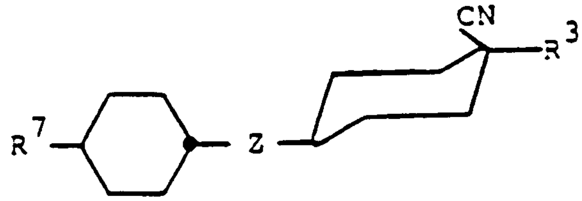

XIII

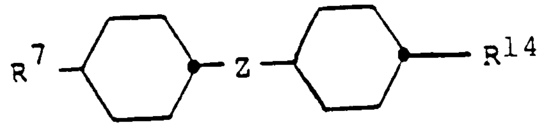

XIV

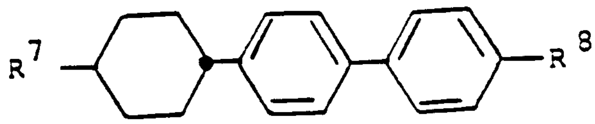

XV

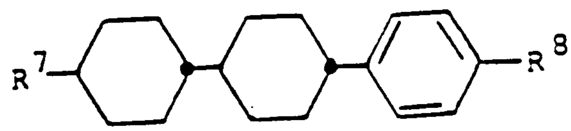

XVI

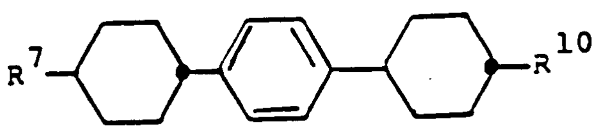

XVII

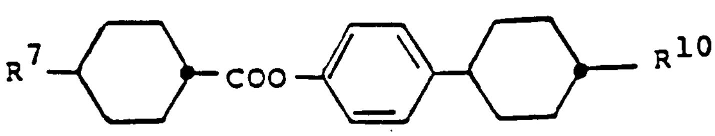

XVIII

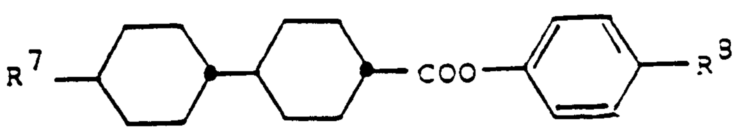

XIX

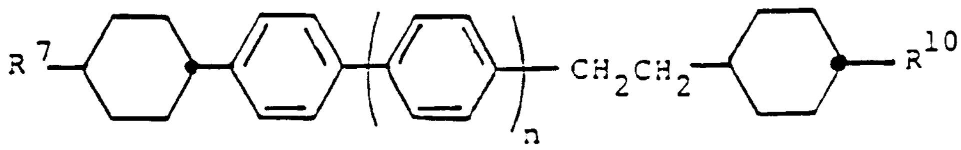

XX

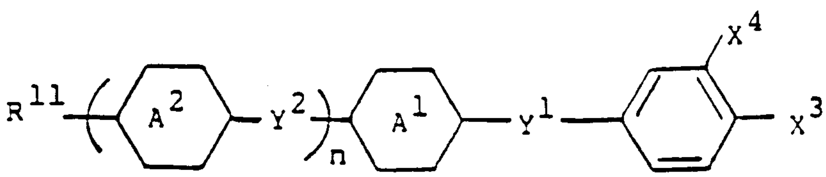

XXI

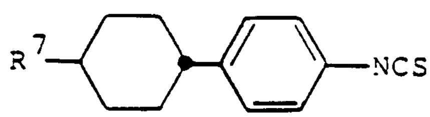

XXII

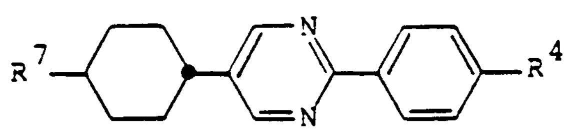

XXIII

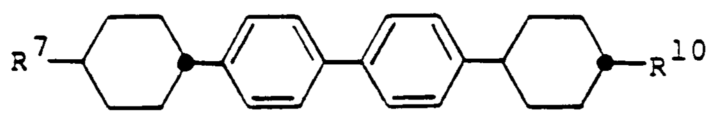

XXIV

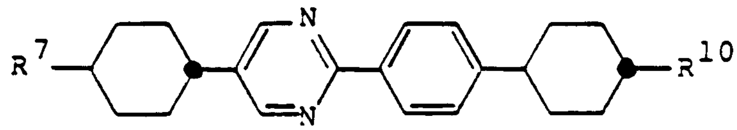

XXV

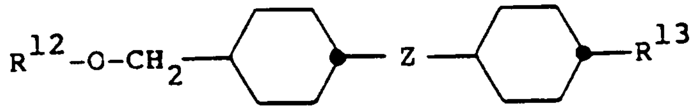

XXVI

worin $R^3$ Alkyl, 3E-Alkenyl oder 4-Alkenyl bedeutet; $R^4$ Alkyl, Cyano oder Fluor darstellt; $R^5$ und $R^6$ Alkyl oder Alkoxy bezeichnen; $R^7$ und $R^{10}$ unabhängig voneinander Alkyl, 1E-Alkenyl, 3E-Alkenyl oder 4-Alkenyl bedeuten; $R^8$ Cyano, Alkyl, 3E-Alkenyl, 4-Alkenyl, Alkoxy, 2E-Alkenyloxy oder 3-Alkenyloxy bezeichnet; $R^9$ Alkoxy, 2E-Alkenyloxy oder 3-Alkenyloxy bedeutet; n für die Zahl O oder 1 steht; Z eine einfache Kovalenzbindung oder $-CH_2CH_2-$ darstellt; $X^3$ Fluor oder Chlor und $X^4$ Wasserstoff, Fluor oder Chlor bezeichnen; $R^{11}$ Alkyl, 3E-Alkenyl, 4-Alkenyl, Alkoxy, 2E-Alkenyloxy oder 3-Alkenyloxy bedeutet; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 $CH_2$-Gruppen durch Stickstoff ersetzt sind, darstellen; $R^{12}$ Alkyl oder 2-Alkenyl bezeichnet; $R^{13}$ 1E-Alkenyl, 3E-Alkenyl, 4-Alkenyl, (2-Alkenyl)oxymethyl, Alkyl oder Cyano bedeutet; und $R^{14}$ Cyano, Alkyl, 1E-Alkenyl, 3E-Alkenyl, 4-Alkenyl, Alkoxy, 2E-Alkenyloxy oder 3-Alkenyloxy bezeichnet.

Der Ausdruck "substituiertes oder unsubstituiertes trans-1,4-Cyclohexylen, in welchem gegebenenfalls 2 nicht benachbarte $CH_2$-Gruppen durch Sauerstoff ersetzt sind", umfasst insbesondere trans-1,4-Cyclohexylen und trans-1,3-Dioxan-2,5-diylsowie Ringe, die mit in Flüssigkristallen üblichen Substituenten, wie Cyano, Methyl, Fluor oder Chlor, substituiert sind, beispielsweise 1-Cyano-trans-1,4-cyclohexylen oder 2-Methyl-trans-1,4-cyclohexylen.

Der Ausdruck "substituiertes oder unsubstituiertes 1,4-Phenylen, in welchem gegebenenfalls 1 oder 2 CH-Gruppen durch Stickstoff ersetzt sind", umfasst insbesondere 1,4-Phenylen, Pyridin-2,5-diyl, Pyrazin-2,5-diyl und Pyrimidin-2,5-diyl, sowie Ringe, die mit in Flüssigkristallen üblichen Substituenten, wie Cyano, Methyl, Fluor oder Chlor substituiert sind, beispielsweise 2-Cyano-1,4-phenylen, 2-Fluor-1,4-phenylen, 2-Chlor-1,4-phenylen oder 2-Methyl-1,4-phenylen.

Bevorzugte Mischungskomponenten mit positiver dielektrischer Anisotropie sind die Cyano- und Halogenverbindungen der Formeln V, VI, VIII, IX, X, XII, XIV, XV, XVI, XIX, XXI und XXIII sowie die Isothiocyanate der Formel XXII. Vorzugsweise enthält das Gesamtgemisch etwa 20-70 Gew.-% insbesondere etwa 25-50 Gew.-% an einer oder mehreren dieser Verbindungen.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, $S_A$ eine smektisch A, N eine nematische und I die isotrope Phase. $V_{10}$ bezeichnet die Spannung für 10% Transmission (Blickrichtung senkrecht zur Plattenoberfläche). $t_{on}$ und $t_{off}$ bezeichnen die Einschaltzeit bzw. die Ausschaltzeit, $\Delta n$ die optische Anisotropie und $\Delta H$ die Schmelzenthalpie.

Beispiel 1

a) Eine Lösung von 9,036 g trans-4-[trans-4-(1E-Propenyl)cyclohexyl]cyclohexancarbonitril in 45 ml Toluol wurde unter Stickstoffbegasung bei 0-4°C innert 55 Minuten tropfenweise mit 39 ml einer 20-proz. (v/v) Lösung von Diisobutylaluminiumhydrid in Toluol versetzt. Nach beendeter Zugabe wurde das Reaktionsgemisch noch 15 Minuten bei 0°C und dann noch 3,5 Stunden bei Raumtemperatur gerührt. Anschliessend wurde die farblose Reaktionslösung unter Kühlung mit einem Eisbad auf 200 ml 1N Schwefelsäure pipettiert und das erhaltene Gemisch einmal mit 400 ml und zweimal mit je 150 ml Methylenchlorid extrahiert. Die vereinigten Methylenchloridphasen wurden zweimal mit je 150 ml Wasser gewaschen, über Natriumsulfat getrocknet, filtriert und eingedampft. Hierbei wurden 10,2 g rohes trans-4-[trans-4-(1E-Propenyl)cyclohexyl]cyclohexancarboxaldehyd als farbloses Oel erhalten.

b) Eine Lösung von 28,5 g Tetrabrommethan in 519 ml Methylenchlorid wurde unter Stickstoffbegasung bei -15°C innert 13 Minuten tropfenweise mit einer Lösung von 45,7 g Triphenylphosphin in 97 ml Methylenchlorid versetzt. Die orange Lösung wurde noch 10 Minuten bei ca. -10°C weitergerührt und dann innert 25 Minuten tropfenweise mit einer Lösung von 10,2 g trans-4-[trans-4-(1E-Propenyl)-cyclohexyl]cyclohexancarboxaldehyd in 112 ml Methylenchlorid versetzt und mit 25 ml Methylenchlorid nachgespült. Das Reaktionsgemisch wurde noch 50 Minuten bei ca. 0°C weiter gerührt und dann auf 3,9

l Hexan gegossen. Das Gemisch wurde 2,5 Stunden bei Raumtemperatur gerührt. Danach wurde der Niederschlag abgenutscht und mit Hexan gewaschen. Das Filtrat wurde im Vakuum eingeengt und der erhaltene, gelbliche, feste Rückstand (33,4 g) in 2 l Hexan suspendiert. Die Suspension wurde 15 Minuten bei Raumtemperatur gerührt und dann 1 Stunde in einem Eisbad gekühlt. Anschliessend wurde der Niederschlag abgenutscht und mit ca. 0,5 l kaltem Hexan gewaschen. Das Filtrat wurde im Vakuum eingeengt. Der farblose, feste Rückstand (19,2 g) wurde an Kieselgel mit Hexan bei 0,4 bar chromatographisch gereinigt. Hierbei wurden 12,7 g trans-4-(2,2-Dibromvinyl)-1-[trans-4-(1E-propenyl)cyclohexyl]-cyclohexan als farblose, feste Substanz erhalten.

c) Eine Lösung von 12,7 g trans-4-(2,2-Dibromvinyl)-1-[trans-4-(1E-propenyl)cyclohexyl]cyclohexan in 500 ml Tetrahydrofuran wurde unter Stickstoffbegasung bei -74 °C innert 30 Minuten tropfenweise mit 49 ml einer 1,6 M Lösung von Butyllithium in Hexan versetzt. Die gelbliche Lösung wurde noch 1 Stunde bei -74 °C weitergerührt, dann auf 500 ml Wasser gegossen und einmal mit 400 ml und zweimal mit je 200 ml Hexan extrahiert. Die vereinigten Hexan-Phasen wurden zweimal mit je 200 ml Wasser gewaschen, über Natriumsulfat getrocknet, filtriert und eingedampft. Hierbei wurden 7,9 g trans-4-Aethinyl-1-[trans-4-(1E-propenyl)cyclohexyl]cyclohexan als farblose Kristalle erhalten. Umkristallisation aus Methylenchlorid/Methanol ergab reines Produkt mit Smp. (C-N) 45, 1 °C, Klp. (N-I) 88,9 °C und $\Delta H = 2{,}323$ kcal/Mol.

In analoger Weise können folgende Verbindungen hergestellt werden:

trans-4-Aethinyl-1-[trans-4-(3-butenyl)cyclohexyl]cyclohexan, Smp. (C-N) 9,0 °C (unterkühlbar bis ca. -40 °C), Klp. (N-I) 64,8 °C, $\Delta H = 2{,}280$ kcal/Mol;
trans-4-Aethinyl-1-[trans-4-(3E-pentenyl)cyclohexyl]cyclohexan, Smp. (C-N) 27,6 °C, Klp. (N-I) 87,7 °C, $\Delta H = 3{,}207$ kcal/Mol;
trans-4-Aethinyl-1-[trans-4(4-pentenyl)cyclohexyl]cyclohexan, Smp.(C-N) 12,7 °C, Klp. (N-I) 41,5 °C, $\Delta H = 4{,}526$ kcal/Mol;
4-Aethinyl-1-[trans-4-(1E-propenyl)cyclohexyl]benzol;
4-Aethinyl-1-[trans-4-(3E-pentenyl)cyclohexyl]benzol;
4-Aethinyl-1-[trans-4-(4-pentenyl)cyclohexyl]benzol;
4′-Aethinyl-4-[trans-4-(1E-propenyl)cyclohexyl]biphenyl;
4′-Aethinyl-4-[trans-4-(3E-pentenyl)cyclohexyl]biphenyl;
4′-Aethinyl-4-[trans-4-(4-pentenyl)cyclohexyl]biphenyl;
4-Aethinyl-1-[trans-4-[trans-4-(1E-propenyl)cyclohexyl]cyclohexyl]benzol;
4-Aethinyl-4′-(3-butenyl)biphenyl;
4-Aethinyl-4′-(4-pentenyl)biphenyl;
4-Aethinyl-2,3-difluor-4′-(3-butenyl)biphenyl;
4-Aethinyl-2,3-difluor-4′-(4-pentenyl)biphenyl;
4-Aethinyl-1-[trans-5-(3-butenyl)-1,3-dioxan-2-yl]benzol;
4-Aethinyl-1-[trans-5-(4-pentenyl)-1,3-dioxan-2-yl]benzol;
4-Aethinyl-4′-[trans-5-(3-butenyl)-1,3-dioxan-2-yl]biphenyl;
4-Aethinyl-1-[trans-4-[trans-5-(3-butenyl)-1,3-dioxan-2-yl]cyclohexyl]benzol;
4-Aethinyl-1-[trans-5-[trans-4-(1E-propenyl)cyclohexyl]-1,3-dioxan-2-yl]benzol;
4-(trans-4-Aethinylcyclohexyl)-1-[trans-5-(1E-propenyl)-1,3-dioxan-2-yl]benzol.

Beispiel 2

Eine Lösung von 1,0 g trans-4-Aethinyl-1-[trans-4-(1E-propenyl)cyclohexyl]cyclohexan in 39 ml Tetrahydrofuran wurde unter Stickstoffbegasung bei -26 °C innert 3 Minuten tropfenweise mit 3,1 ml einer 1,6 M Lösung von Butyllithium in Hexan versetzt. Die gelbliche Lösung wurde noch 30 Minuten bei -20 °C weiter gerührt, dann mit 4,3 ml Hexamethylphosphorsäuretriamid und anschliessend mit 0,696 g Propylbromid versetzt. Das Reaktionsgemisch wurde noch 3,5 Stunden bei Raumtemperatur gerührt, dann auf 65 ml Wasser gegossen und anschliessend einmal mit 50 ml und zweimal mit je 40 ml Hexan extrahiert. Die vereinigten Hexan-Phasen wurden zweimal mit je 30 ml Wasser gewaschen, über Natriumsulfat getrocknet, filtriert und eingedampft. Das gelbliche, nematische Rohprodukt (1,186 g) wurde an Kieselgel mit Hexan bei 0,4 bar chromatographisch gereinigt. Hierbei wurden 1,088 g trans-4-(1-Pentinyl)-1-[trans-4-(1E-propenyl)-cyclohexyl]cyclohexan als farblose, nematische Substanz erhalten. Umkristallisation aus Diäthyläther/Methanol ergab 0,805 g reines Produkt mit Smp. (C-N) 28,1 °C, Klp. (N-I) 68,6 °C und $\Delta H = 5{,}602$ kcal/Mol.

In analoger Weise können folgende Verbindungen hergestellt werden:

trans-4-(1-Propinyl)-1-[trans-4-(1E-propenyl)cyclohexyl]cyclohexan, Smp. (C-N) 69,2 °C, Klp. (N-I) 117,7 °C,

ΔH = 2,310 kcal/Mol;
trans-4-(1-Butinyl)-1-[trans-4-(1E-propenyl)cyclohexyl]cyclohexan, Smp. (C-N) 26,8 °C, Klp. (N-I) 82,1 °C, ΔH = 4,430 kcal/ Mol;
trans-4-(1-Propinyl)-1-[trans-4-(3-butenyl)cyclohexyl]cyclohexan,Smp. (C-N) 9,2 °C, Klp. (N-I) 71,5 °C, ΔH = 3,714 kcal/Mol;
trans-4-(1-Butinyl)-1-[trans-4-(3-butenyl)cyclohexyl]cyclohexan, Smp. (C-N) 6,8 °C, Klp. (N-I) 52,5 °C, ΔH = 4,522 kcal/Mol;
trans-4-(1-Propinyl)-1-[trans-4-(3E-pentenyl)cyclohexyl]cyclohexan, Smp. (C-N) 47,5 °C, Klp. (N-I) 105,5 °C, ΔH = 4,594 kcal/Mol;
trans-4-(1-Butinyl)-1-[trans-4-(3E-pentenyl)cyclohexyl]cyclohexan, Smp. (C-N) 22,8 °C, Klp. (N-I) 86,5 °C, ΔH = 4,406 kcal/Mol;
trans-4-(1-Pentinyl)-1-[trans-4-(3E-pentenyl)cyclohexyl]cyclohexan, Smp. (C-N) 35,8 °C, Klp. (N-I) 77,9 °C, ΔH = 4,160 kcal/Mol;
trans-4-(1-Hexinyl)-1-[trans-4-(3E-pentenyl)cyclohexyl]cyclohexan, Smp. (C-N) 34,5 °C, Klp. (N-I) 55,5 °C, ΔH = 5,058 kcal/Mol;
trans-4-(1-Propinyl)-1-[trans-4-(4-pentenyl)cyclohexyl]cyclohexan, Smp. (C-N) 28,8 °C, Klp. (N-I) 61,3 °C, ΔH = 5,418 kcal/Mol;
trans-4-(1-Butinyl)-1-[trans-4-(4-pentenyl)cyclohexyl]cyclohexan,Smp. (C-N) 6,6 °C, Klp. (N-I) 44,5 °C, ΔH = 4,168 kcal/Mol;
trans-4-(1-Pentinyl)-1-[trans-4-(4-pentenyl)cyclohexyl]cyclohexan, Smp. (C-N) -11,0 °C, Klp. (N-I) 58,9 °, ΔH = 3,366 kcal/Mol;
4-(1-Propinyl)-1-[trans-4-(1E-propenyl)cyclohexyl]benzol, Smp. (C-N) 69,4 °C, Klp. (N-I) 90,7 °C, ΔH = 4,509 kcal/Mol;
4-(1-Propinyl)-1-[trans-4-(3E-pentenyl)cyclohexyl]benzol, Smp. (C-N) 48,6 °C, Klp. (N-I) 82,5 °C, ΔH = 3,127 kcal/Mol;
4-(1-Propinyl)-1-[trans-4-(4-pentenyl)cyclohexyl]benzol, Smp. (C-N) 13,9 °C, Klp. (N-I) 31,2 °C, ΔH = 4,127 kcal/Mol;
4'-(1-Propinyl)-4-[trans-4-(1E-propenyl)cyclohexyl]biphenyl, Smp. (C-N) 189 °C, Klp. (N-I) > 300 °C (Zersetzung), ΔH = 5,443 kcal/Mol;
4'-(1-Propinyl)-4-[trans-4-(3E-pentenyl)cyclohexyl]biphenyl, Smp. ($C-S_A$) 144,8 °C, Uebergang $S_A$-N 181,5 °C, Klp. (N-I) > 266 °C (Zersetzung), ΔH = 4,490 kcal/Mol;
4-(1-Propinyl)-1-[trans-4-[trans-4-(1E-propenyl)cyclohexyl]cyclohexyl]benzol;
4-(1-Propinyl)-4' -(3-butenyl)biphenyl;
4-(1-Propinyl)-4'-(4-pentenyl)biphenyl;
4-(1-Propinyl)-2,3-difluor-4'-(3-butenyl)biphenyl;
4-(1-Propinyl)-2,3-difluor-4'-(4-pentenyl)biphenyl;
4-(1-Propinyl)-1-[trans-5-(3-butenyl)-1,3-dioxan-2-yl]benzol;
4-(1-Propinyl)-1-[trans-5-(4-pentenyl)-1,3-dioxan-2-yl]benzol, Smp. (C-I) 68 °C;
4-(1-Propinyl)-4'-[trans-5-(3-butenyl)-1,3,dioxan-2-yl]biphenyl, Smp. (C-N) 116,1 °C, S-N 91 °C, Klp. (N-I) 235,7 °C, ΔH = 4,971 kcal/Mol;
4-(1-Propinyl)-1-[trans-4-[trans-5-(3-butenyl)-1,3-dioxan-2-yl]-cyclohexyl]benzol, Smp. (C-N) 131,5 °C, Klp. (N-I) 212,3 °C, ΔH = 7,235 kcal/Mol;
4-(1-Propinyl)-1-[trans-5-[trans-4-(1E-propenyl)cyclohexyl]-1,3-dioxan-2-yl]benzol;
4-[trans-4-(1-Propinyl)cyclohexyl]-1-[trans-5-(1E-propenyl)-1,3-dioxan-2-yl]benzol.

Beispiel 3

Aus 4-(trans-4-Pentylcyclohexyl)benzonitril und je einer Verbindung der Formel I wurden mehrere binäre Gemische hergestellt und der Einfluss auf Klärpunkt, $V_{10}$, $t_{on}$, $t_{off}$ und Δn untersucht. $V_{10}$, $t_{on}$ und $t_{off}$ wurden in einer TN-Zelle mit 8 µm Plattenabstand bei 22 °C gemessen, wobei als Betriebsspannung der 2,5-fache Wert von $V_{10}$ gewählt wurde. Δn wurde bei 22 °C gemessen. Die verwendeten Komponenten der Formel I, deren Konzentration im binären Gemisch und die gemessenen Daten sind in Tabelle 1 aufgeführt. Zum Vergleich sind die entsprechenden Werte für reines 4-(trans-4-Pentylcyclohexyl)benzonitril angegeben.

Tabelle 1

Binäre Gemische mit 4-(trans-4-Pentylcyclohexyl)benzonitril

| Komponente der Formel I, Konzentration in Gew.-% | Klp.(N-I) [°C] | $V_{10}$ [Volt] | $t_{on}$ [ms] | $t_{off}$ [ms] | Δn |
|---|---|---|---|---|---|
| [100% 4-(trans-4-Pentylcyclohexyl)benzonitril] | 54,6 | 1,62 | 30 | 42 | 0,120 |
| 10% trans-4-Aethinyl-1-[trans-4-(1E-propenyl)-cyclohexyl]cyclohexan | 60,3 | 1,69 | 22 | 37 | 0,123 |
| 20% trans-4-Aethinyl-1-[trans-4-(1E-propenyl)-cyclohexyl]cyclohexan | 66,0 | 1,89 | 20 | 35 | 0,122 |
| 10% trans-4-(1-Propinyl)-1-[trans-4-(1E-propenyl)-cyclohexyl]cyclohexan | 56,2 | 1,70 | 20 | 36 | 0,122 |
| 20% trans-4-(1-Propinyl)-1-[trans-4-(1E-propenyl)-cyclohexyl]cyclohexan | 58,9 | 1,79 | 21 | 35 | 0,119 |
| 10% trans-4-(1-Propinyl)-1-[trans-4-(3E-pentenyl)-cyclohexyl]cyclohexan | 57,9 | 1,62 | 22 | 37 | 0,122 |
| 50% trans-4-(1-Propinyl)-1-[trans-4-(3E-pentenyl)-cyclohexyl]cyclohexan | 73,1 | 2,53 | 20 | 34 | 0,114 |
| 10% trans-4-(1-Butinyl)-1-[trans-4-(1E-propenyl)-cyclohexyl]cyclohexan | 53,9 | 1,62 | 20 | 36 | 0,118 |

## Tabelle 1

Binäre Gemische mit 4-(trans-4-Pentylcyclohexyl)benzonitril
(FORTSETZUNG)

| Komponente der Formel I, Konzentration in Gew.-% | Klp.(N-I) [°C] | $V_{10}$ [Volt] | $t_{on}$ [ms] | $t_{off}$ [ms] | Δn |
|---|---|---|---|---|---|
| 20% trans-4-(1-Butinyl)-1-[trans-4-(1E-propenyl)-cyclohexyl]cyclohexan | 54,2 | 1,69 | 20 | 36 | 0,115 |
| 10% 4-(1-Propinyl)-1-[trans-4-(1E-propenyl)cyclohexyl]benzol | 57,0 | 1,60 | 21 | 36 | 0,128 |
| 50% 4-(1-Propinyl)-1-[trans-4-(1E-propenyl)cyclohexyl]benzol | 69,1 | 2,29 | 16 | 27 | >0,140 |
| 10% 4-(1-Propinyl)-1-[trans-4-(3E-pentenyl)cyclohexyl]benzol | 61,7 | 1,73 | 21 | 36 | 0,136 |
| 10% 4-(1-Propinyl)-1-[trans-4-(4-pentenyl)cyclohexyl]benzol | 52,7 | 1,47 | 22 | 39 | 0,122 |
| 10% 4'-(1-Propinyl)-4-[trans-4-(3E-pentenyl)cyclohexyl]benzol | 71,3 | 1,73 | 25 | 40 | 0,141 |

## Patentansprüche

**1.** Verbindungen der allgemeinen Formel

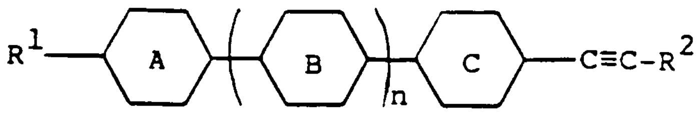

I

worin n für die Zahl O oder 1 steht; die Ringe A, B und C unabhängig voneinander trans-1,4-Cyclohexylen, trans-1,3-Dioxan-2,5-diyl oder unsubstituiertes oder fluorsubstituiertes 1,4-Phenylen darstellen; $R^1$ 3E-Alkenyl mit 4 bis 12 Kohlenstoffatomen, 4-Alkenyl mit 5 bis 12 Kohlenstoffatomen oder, wenn Ring A trans-1,4-Cyclohexylen oder trans-1,3-Dioxan-2,5-diyl darstellt, auch 1E-Alkenyl mit 2 bis 12 Kohlenstoffatomen bezeichnet; und $R^2$ Wasserstoff oder $C_1$-$C_{10}$-Alkyl bedeutet.

**2.** Verbindungen nach Anspruch 1 gekennzeichnet durch eine der allgemeinen Formeln

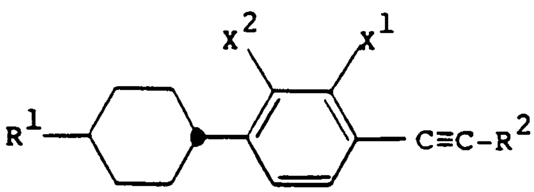

I-1

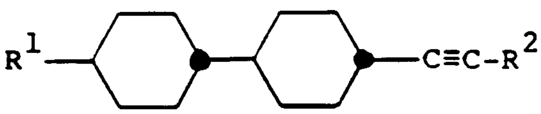

I-2

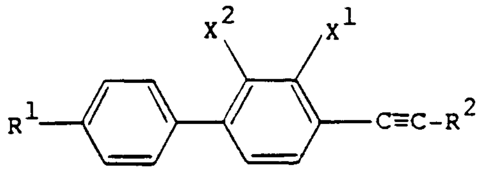

I-3

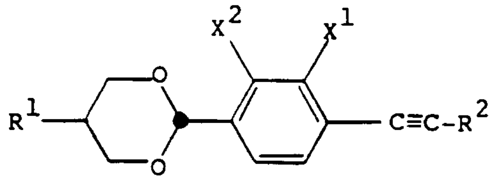

I-4

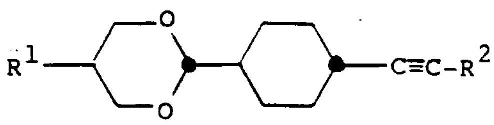

I-5

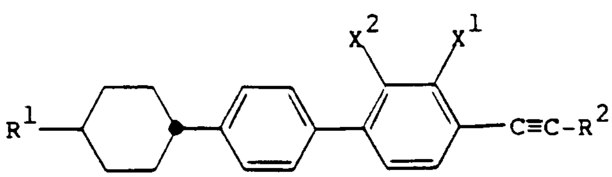

I-6

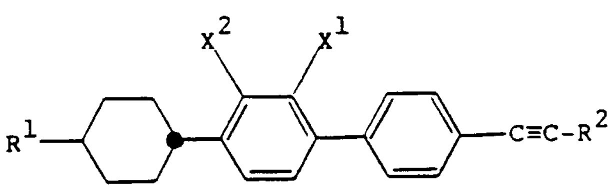

I-7

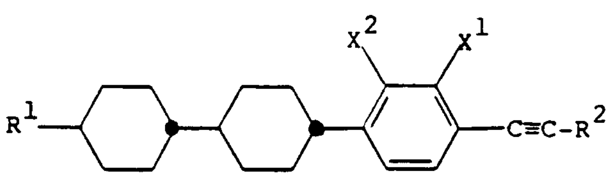

I-8

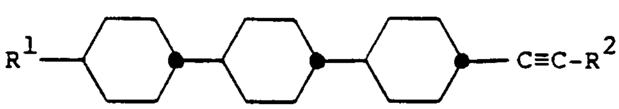

I-9

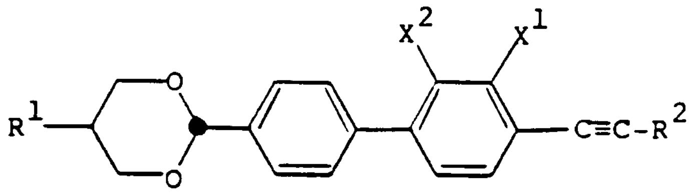

I-10

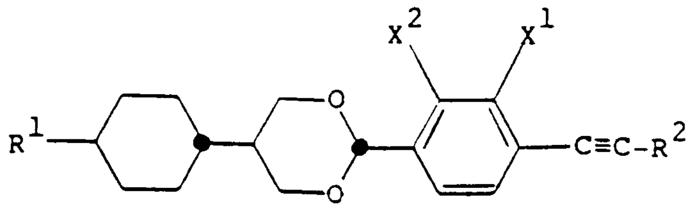

I-11

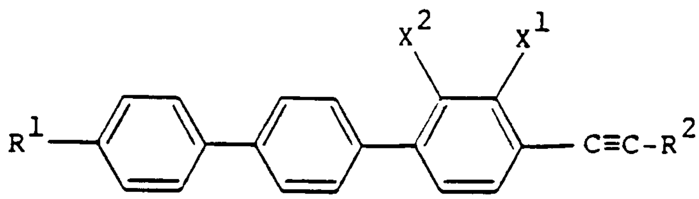

I-12

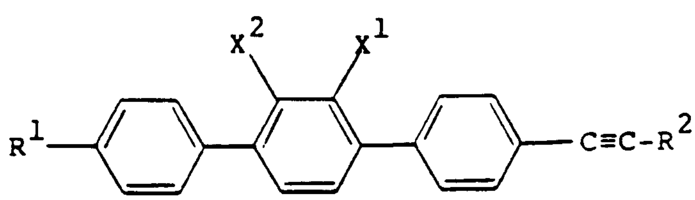

I-13

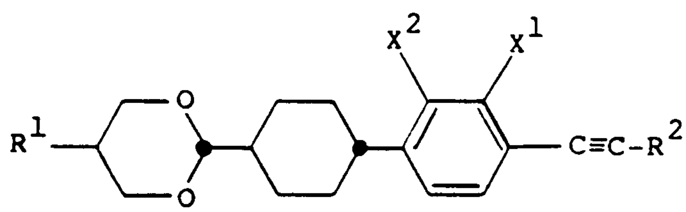

I-14

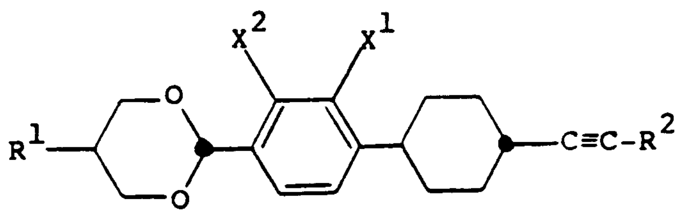

I-15

worin $X^1$ und $X^2$ unabhängig voneinander Wasserstoff oder Fluor darstellen; $R^2$ Wasserstoff oder $C_1$-$C_{10}$-Alkyl bedeutet; und $R^1$ 3E-Alkenyl mit 4 bis 12 Kohlenstoffatomen, 4-Alkenyl mit 5 bis 12 Kohlenstoffatomen oder in den Formeln I-1, I-2, I-4, I-5, I-6, I-7, I-8, I-9, I-10, I-11, I-14 und I-15 auch 1E-Alkenyl mit 2 bis 12 Kohlenstoffatomen bezeichnet.

**3.** Verbindungen nach Anspruch 1 oder 2, dadurch gekennzeichnet, dass $R^1$ ein geradkettiger Rest und $R^2$ Wasserstoff oder ein geradkettiger Rest ist.

**4.** Verbindungen nach einem der Ansprüche 1 bis 3, dadurch gekennzeichnet, dass $R^1$ höchstens 7, vorzugsweise höchstens 5 Kohlenstoffatome aufweist.

**5.** Verbindungen nach einem der Ansprüche 1 bis 4, dadurch gekennzeichnet, dass $R^2$ Wasserstoff oder $C_1$-$C_5$-Alkyl, vorzugsweise Wasserstoff oder $C_1$-$C_3$-Alkyl bedeutet.

**6.** Verbindungen nach einem der Ansprüche 1 bis 5, dadurch gekennzeichnet, dass $R^1$ 3-Butenyl, 3E-Pentenyl, 4-Pentenyl oder an trans-1,4-Cyclohexylen oder trans-1,3-Dioxan-2,5-diyl auch 1E-Propenyl.

**7.** Flüssigkristallines Gemisch mit mindestens 2 Komponenten, dadurch gekennzeichnet, dass mindestens eine Komponente eine Verbindungen der in Anspruch 1 definierten Formel I ist.

**8.** Flüssigkristallines Gemisch nach Anspruch 7, dadurch gekennzeichnet, dass es eine oder mehrere Verbindungen der Formel I und eine oder mehrere Verbindungen mit positiver dielektrischer Anisotropie enthält.

**9.** Verfahren zur Herstellung der Verbindungen der in Anspruch 1 definierten allgemeinen Formel I, dadurch gekennzeichnet, dass man

a) zur Herstellung der Verbindungen der Formel I, worin $R^2$ Wasserstoff bedeutet, eine Verbindung der allgemeinen Formel

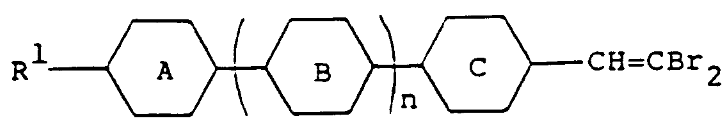

II

worin n, $R^1$ und die Ringe A, B und C die in Formel I gegebenen Bedeutungen haben,

mit Alkyllithium umsetzt, oder

b) zur Herstellung der Verbindungen der Formel I, worin $R^2$ $C_1$-$C_{12}$-Alkyl bedeutet, eine Verbindung der Formel I, worin $R^2$ Wasserstoff bedeutet, alkyliert.

**10.** Verwendung der Verbindungen der in Anspruch 1 definierten Formel I für elektro-optische Zwecke.

## Claims

**1.** Compounds of the general formula

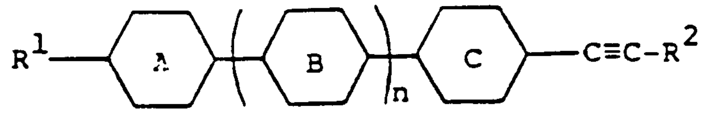

I

wherein n stands for the number 0 or 1; rings A, B and C each independently represent trans-1,4-cyclohexylene, trans-1,3-dioxane-2,5-diyl or unsubstituted or fluorosubstituted 1,4-phenylene; $R^1$ denotes 3E-alkenyl with 4 to 12 carbon atoms, 4-alkenyl with 5 to 12 carbon atoms or, when ring A represents trans-1,4-cyclohexylene or trans-1,3-dioxane-2,5-diyl, also 1E-alkenyl with 2 to 12 carbon atoms; and $R^2$ signifies hydrogen or $C_1$-$C_{10}$-alkyl.

**2.** Compounds according to claim 1 characterized by one of the general formulae

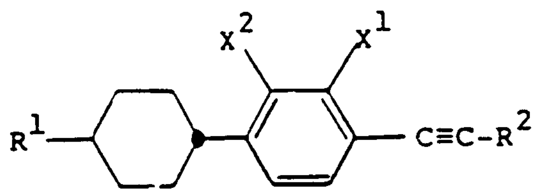

I-1

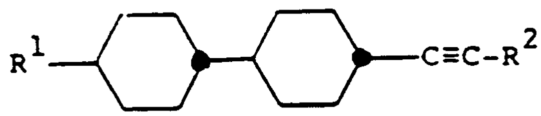

I-2

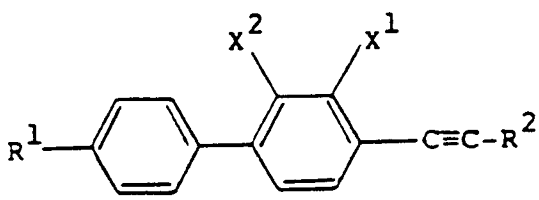

I-3

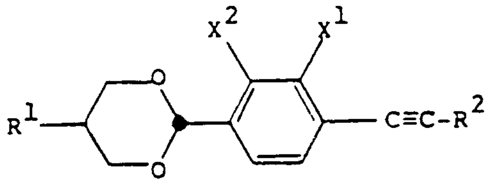

I-4

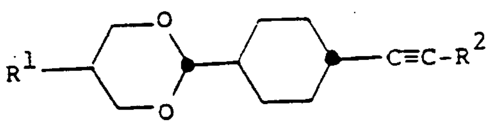

I-5

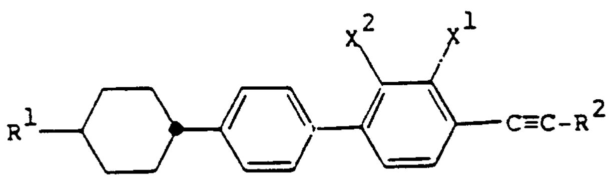

I-6

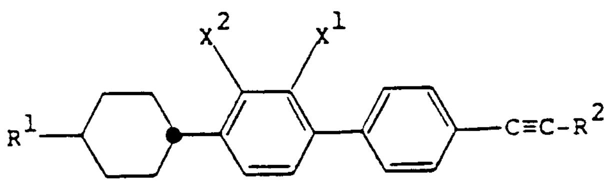

I-7

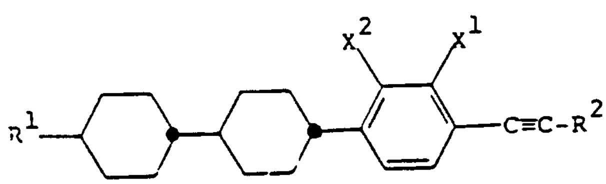

I-8

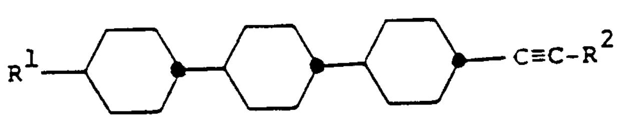

I-9

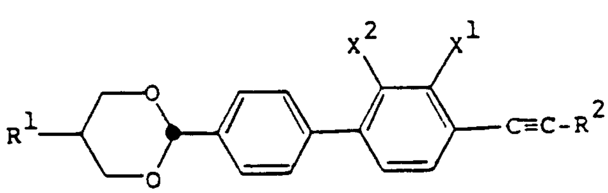

I-10

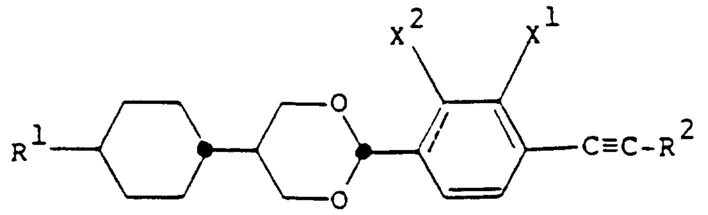

I-11

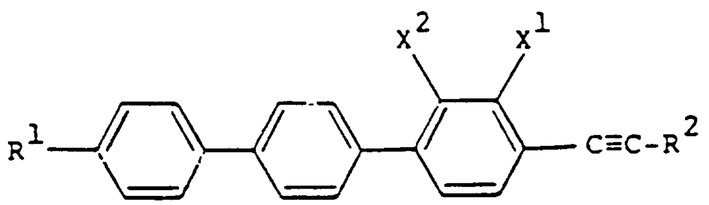

I-12

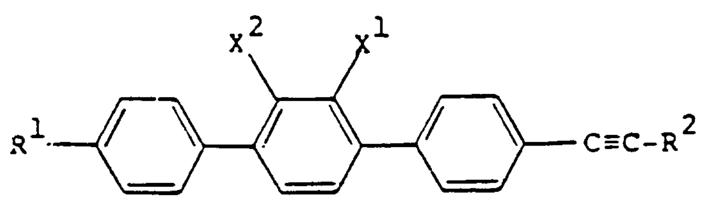

I-13

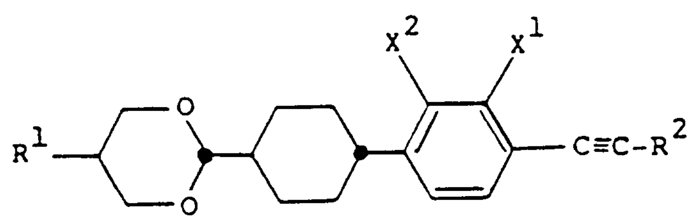

I-14

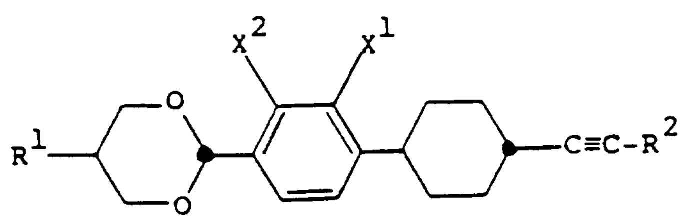

I-15

wherein $X^1$ and $X^2$ each independently represent hydrogen or fluorine; $R^2$ signifies hydrogen or $C_1$-$C_{10}$-alkyl; and $R^1$ denotes 3E-alkenyl with 4 to 12 carbon atoms, 4-alkenyl with 5 to 12 carbon atoms or in formulae I-1, I-2, I-4, I-5, I-6, I-7, I-8, I-9, I-10, I-11, I-14 and I-15 also 1E-alkenyl with 2 to 12 carbon atoms.

**3.** Compounds according to claim 1 or 2, characterized in that $R^1$ is a straight-chain residue and $R^2$ is hydrogen or a straight-chain residue.

**4.** Compounds according to any one of claims 1 to 3, characterized in that $R^1$ has a maximum of 7, preferably a maximum of 5, carbon atoms.

**5.** Compounds according to any one of claims 1 to 4, characterized in that $R^2$ signifies hydrogen or $C_1$-$C_5$-alkyl, preferably hydrogen or $C_1$-$C_3$-alkyl.

**6.** Compounds according to any one of claims 1 to 5, characterized in that $R^1$ signifies 3-butenyl, 3E-pentenyl, 4-pentenyl or on trans-1,4-cyclohexylene or trans-1,3-dioxane-2,5-diyl also 1E-propenyl.

**7.** A liquid crystalline mixture having at least 2 components, characterized in that at least one component is a compound of formula I defined in claim 1.

**8.** A liquid crystalline mixture according to claim 7, characterized in that it contains one or more compounds of formula I and one or more compounds having positive dielectric anisotropy.

**9.** A process for the manufacture of the compounds of general formula I defined in claim 1, characterized by

a) for the manufacture of the compounds of formula I in which $R^2$ signifies hydrogen, reacting a compound of the general formula

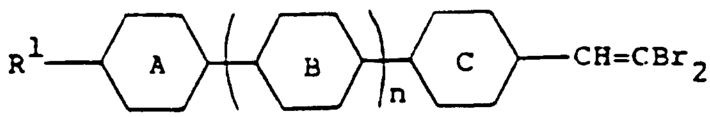

II

wherein n, $R^1$ and rings A, B and C have the significances given in formula I,

with an alkyllithium, or

b) for the manufacture of the compounds of formula I in which $R^2$ signifies $C_1$-$C_{12}$-alkyl, alkylating a compound of formula I in which $R^2$ signifies hydrogen.

**10.** The use of the compounds of formula I defined in claim 1 for electro-optical purposes.

**Revendications**

**1.** Composés de formule générale

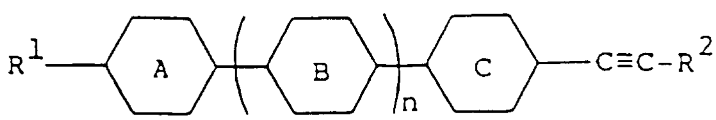

I

où n est égal à 0 ou 1 ; les cycles A, B et C représentent, indépendamment les uns des autres, le trans-1,4-cyclohexylène, le trans-1,3-dioxane-2,5-diyle ou le 1,4-phénylène non substitué ou substitué par le fluor ; $R^1$ représente un 3E-alcényle ayant 4 à 12 atomes de carbone, un 4-alcényle ayant 5 à 12 atomes de carbone ou, lorsque le cycle A représente le trans-1,4-cyclohexylène ou le trans-1,3-dioxane-2,5-diyle, désigne également un 1E-alcényle ayant 2 à 12 atomes de carbone ; et $R^2$ représente l'hydrogène ou un alkyle en $C_1$-$C_{10}$.

**2.** Composés selon la revendication 1, caractérisés par l'une des formules générales

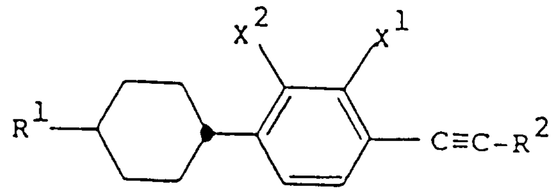

I-1

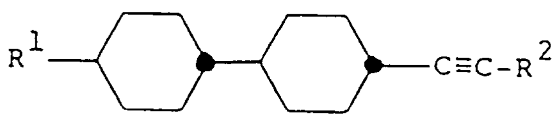

I-2

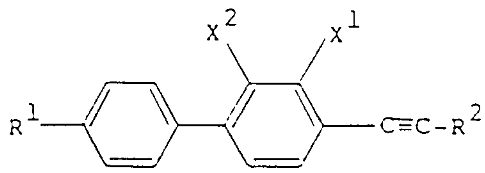

I - 3

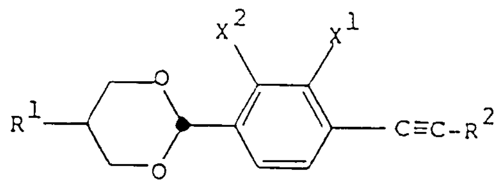

I - 4

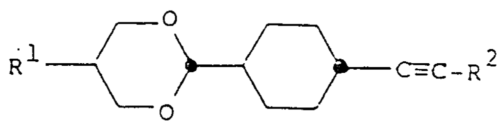

I - 5

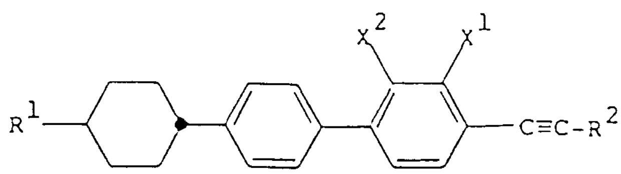

I - 6

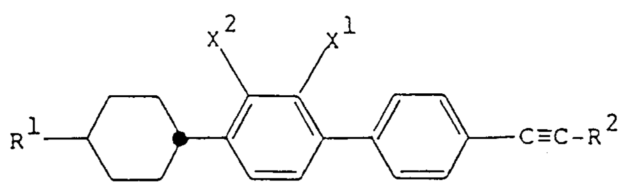

I - 7

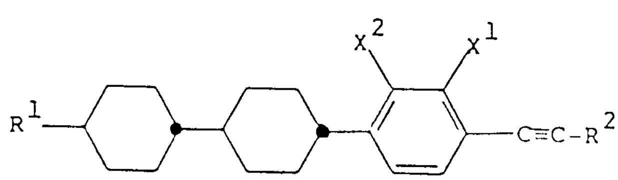

I - 8

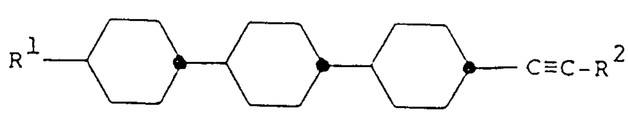

I - 9

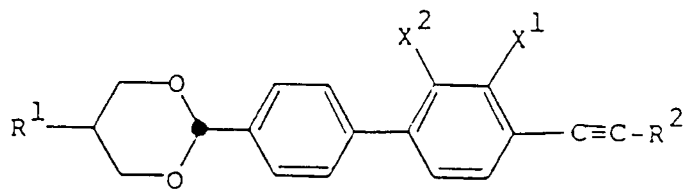

I-10

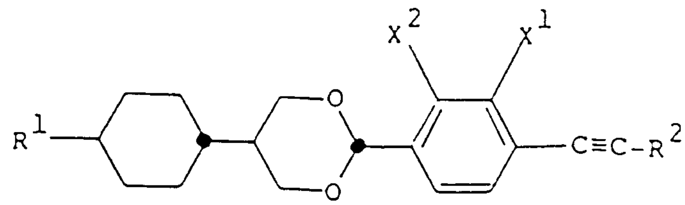

I-11

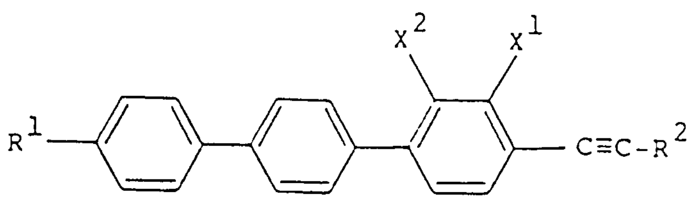

I-12

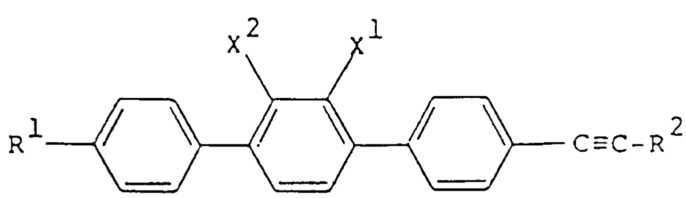

I-13

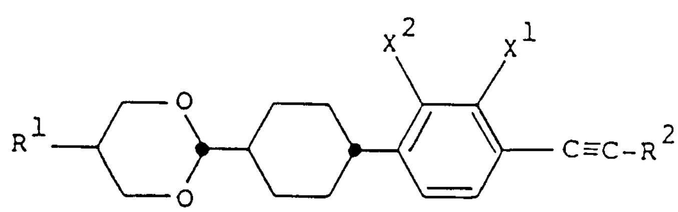

I-14

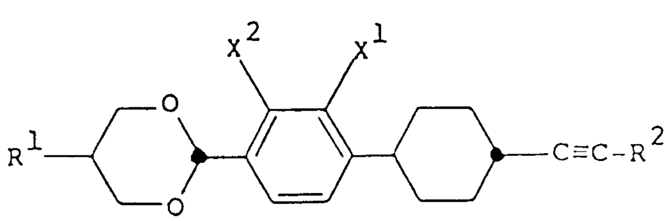

I-15

où $X^1$ et $X^2$ représentent, indépendamment l'un de l'autre, l'hydrogène ou le fluor ; $R^2$ représente l'hydrogène ou un alkyle en $C_1$-$C_{10}$ ; et $R^1$ représente un 3E-alcényle ayant 4 à 12 atomes de carbone, un 4-alcényle ayant 5 à 12 atomes de carbone ou désigne dans les formules I-1, I-2, I-4, I-5, I-6, I-7, I-8, I-9, I-10, I-11, I-14 et I-15 également un 1E-alcényle ayant 2 à 12 atomes de carbone.

**3.** Composés selon la revendication 1 ou 2, caractérisés en ce que $R^1$ est un reste linéaire et $R^2$ l'hydrogène ou un reste linéaire.

**4.** Composés selon l'une des revendications 1 à 3, caractérisés en ce que $R^1$ présente au plus 7, de préférence, au plus 5 atomes de carbone.

**5.** Composés selon l'une des revendications 1 à 4, caractérisés en ce que $R^2$ représente l'hydrogène ou un alkyle en $C_1$-$C_5$, de préférence, l'hydrogène ou un alkyle en $C_1$-$C_3$.

**6.** Composés selon l'une des revendications 1 à 5, caractérisés en ce que $R^1$ représente le 3-buténylе, le 3E-penténylе, le 4-penténylе ou, lorsque le cycle A représente le trans-1,4-cyclohexylène ou le trans-1,3-dioxane-2,5-diyle, également le 1E-propényle.

**7.** Mélange à cristaux liquides comportant au moins 2 composants, caractérisé en ce qu'au moins un composant est un composé de formule I définie dans la revendication 1.

**8.** Mélange à cristaux liquides selon la revendication 7, caractérisé en ce qu'il contient un ou plusieurs composés de formule I et un ou plusieurs composés présentant une anisotropie diélectrique positive.

**9.** Procédé pour la préparation des composés de formule générale I définie dans la revendication 1, caractérisé

a) en ce que, pour la préparation des composés de formule I où $R^2$ représente l'hydrogène, l'on fait réagir un composé de formule générale

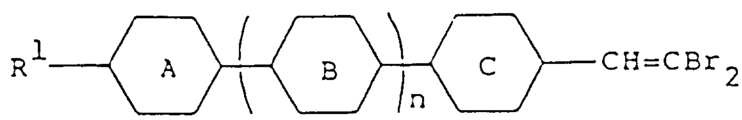

II

où n, $R^1$ et les cycles A, B et C ont les significations données pour la formule I, avec un alkyllithium ou

b) en ce que, pour la préparation des composés de formule I où $R^2$ représente un alkyle en $C_1$-$C_{12}$, on alcoyle un composé de formule I où $R^2$ représente l'hydrogène.

**10.** Utilisation des composés de formule I définie dans la revendication 1 à des fins électro-optiques.